# EUROPÄISCHE PATENTSCHRIFT

(11) **EP 2 092 051 B1**
(45) Veröffentlichungstag und Bekanntmachung des Hinweises auf die Patenterteilung: **27.02.2019**
(21) Anmeldenummer: 07856054.7
(22) Anmeldetag: 06.12.2007
(51) Int. Cl.: C12M 1/113

(54) **FERMENTER**
BIOREACTOR
FERMENTEUR

(30) Priorität: 07.12.2006 DE 102006058030; 22.03.2007 DE 102007014417
(43) Veröffentlichungstag der Anmeldung: 26.08.2009
(73) Patentinhaber: Horstmann, Bernhard, 32545 Bad Oynhausen (DE)
(72) Erfinder: SCHLÜTER, Thomas, 71093 Weil im Schönbuch (DE); HEITLAND, Uwe, 32545 Bad Oeynhausen (DE)
(74) Vertreter: Rieke, Andreas
(86) Internationale Anmeldenummer: PCT/DE2007/002190
(87) Internationale Veröffentlichungsnummer: WO 2008/067800

(56) Entgegenhaltungen:
- WO-A-2006/079227
- AT-B- 405 185
- DE-A1- 3 309 356
- DE-A1- 4 416 521

## Beschreibung

Die Erfindung betrifft einen Fermenter, insbesondere einen Propfenstromfermenter, der ein Langachsrührwerk für ein Durchmischen einer auszugasenden Biomasse hoher Trockensubstanz in einem Gärraum aufweist, bei dem eine Welle des Rührwerks ausschließlich in zwei gegenüberliegenden, stirnseitigen Wänden des Gärraums gelagert ist und bei dem wenigstens ein freies Ende des Rührwerks außerhalb des Gärraums an einen Antrieb angeschlossen ist.

Die Verwendung solcher Fermenter ist in Biogasanlagen bekannt und bewährt. Dabei dienen die eingangs genannten Fermenter der Vergärung von Bioabfällen, Grüngut, Speiseresten oder dergleichen, die einen hohen Anteil an Trockenmasse aufweisen, beispielsweise von mehr als 40%, ohne dass ein Güllezusatz erfolgt. Die auftretende Fermentierung ist eine mesophile oder thermophile Trockenvergärung bei moderaten Temperaturen, beispielsweise zwischen 37°C und 41°C oder 50°C und 60°C.

Darüber hinaus kann ein Propfenstromfermenter kontinuierlich beschickt werden, gleich bedeutend mit einem großen Eintrag an zu vergärender Biomasse, was eine hohe Gasausbeute zur Folge hat. Hierbei sorgt das Rührwerk für eine optimale Entgasung und eine gleichmäßige Temperaturverteilung und der auftretende Propfenstrom für eine definierte Verweilzeit des Substrats in dem Fermenter.

Aus der DE 44 16 521 A1 ist ein gattungsgemäßer Fermenter zur Vergärung biogener Dickstoff-Abfälle für die Gewinnung von Biogas bekannt, der einen zylindrischen oder halbzylindrischen, liegenden Reaktorbehälter mit gewölbten Stirnseiten aufweist, in denen ein von außerhalb des Reaktorbehälters angetriebenes Rührwerk gelagert ist. Ein solcher Reaktorbehälter nach Art eines Autoklaven aus Metall ist, bei entsprechender Größe, vergleichsweise teuer und es bedarf darüber hinaus einer besonderen Lagerung.

Entsprechendes gilt für die aus der WO 2006/079227 A1 und der AT 405 185 B bekannten Fermenter mit zylindrischen Reaktorbehältern.

Propfenstromfermenter der in Rede stehenden Art sind vergleichsweise konstruktiv aufwendig und bedürfen regelmäßig einer Vielzahl von Nebengebäuden, beispielsweise Betriebsgebäuden, Gasspeicher und dergleichen mehr.

Entsprechend hoch sind die Investitionskosten solcherart betriebener Biogasanlagen.

Vor diesem technischen Hintergrund macht die Erfindung es sich zur Aufgabe, einen Fermenter zur Verfügung zu stellen, der in kurzer Bauzeit einfach und damit preiswert erstellbar ist und der bei einer hohen Gasleistung sehr wartungsfreundlich ist.

Gelöst wird diese technische Problematik bei einem Fermenter, insbesondere einem Propfenstromfermenter, mit einem Langachsrührwerk für ein Durchmischen einer auszugasenden Biomasse hoher Trockensubstanz in einem Gärraum, bei dem eine Welle des Rührwerks ausschließlich in zwei gegenüberliegenden, stirnseitigen Wänden des Gärraums gelagert ist und dass wenigstens ein freies Ende des Rührwerks außerhalb des Gärraums an einen Antrieb angeschlossen ist, gemäß des Anspruchs 1 durch die Maßnahmen, dass das Drehmoment des Antriebs von einer aufgehenden, stirnseitigen Wand des Gärraums entkoppelt in ein Fundament eingeleitet wird oder dass das Drehmoment des Antriebs über eine Drehmomentstütze von dem Lager abgesetzt in eine aufgehenden, stirnseitige Wand des Gärraums eingeleitet wird.

Durch die ausschließliche Lagerung des Langachsrührwerks in den stirnseitigen Wänden bildet sich ein ungestörter Propfenstrom aus, der einen gleichmäßigen Massedurchsatz und eine gleichmäßige Temperaturverteilung in dem auszugasenden Substrat gewährleistet. Totwässer an einer mittigen, zusätzlichen Lagerstelle der Welle des Rührwerks sind vermieden, da erfindungsgemäß auf ein solches, vollständig im Gärsubstrat mitlaufendes und deshalb anfälliges Mittellager verzichtet ist.

Die Anordnung des Antriebs außerhalb des Gärraums ermöglicht in einfacher Weise eine Revision desselben, ohne dass ein regelmäßig gefährliches Betreten des Gärraums nötig ist. Ein solches Betreten des Gärraums eines Fermenters nach der Erfindung wird somit einen Ausnahmefall darstellen.

Bei dem erfindungsgemäßen Fermenter ist vorgesehen, dass das Drehmoment des Antriebs von einer aufgehenden stirnseitigen Wand des Gärraums entkoppelt in ein Fundament eingeleitet wird. Entsprechend schwach dimensioniert kann diese stirnseitige Wand dann auch ausgebildet sein.

Alternativ kann vorgesehen werden, dass das Drehmoment des Antriebs über eine Drehmomentstütze von dem Lager abgesetzt in eine aufgehenden, stirnseitige Wand des Gärraums eingeleitet wird.

Beide Alternativen erlauben es, die hohe Antriebsleistung von rund 30kW bei einem Drehmoment von etwa 100.000 Nm sicher aufzufangen, ohne dass es in unmittelbarer Umgebung des Durchbruchs für das Lager bzw. die Welle zu einer Schwächung der Wand durch eine Vielzahl von in dieser eingelassenen Befestigungsbolzen, Schwerlastdübeln oder dergleichen kommt.

Darüber hinaus ist regelmäßig vorgesehen, dass beide Lager des Rührwerks von außerhalb des Gärraums zugänglich und austauschbar sind, womit bei einer Revision wie auch in einem Schadensfall, naturgemäß nach ausreichender Absenkung des Substratpegels in dem Gärraum, ein Betreten des Gärraums nicht von Nöten ist.

Erfindungsgemäist vorgesehen, dass eine zentrale Welle Rührwerks, aus mehreren axialen Abschnitten zusammengesetzt, stirnwandseitig jeweils einen Abschnitt reduzierten Durchmessers aufweist. Dementsprechend können die Lage in den stirnseitigen Wänden einen geringen Durchmesser auch nur aufweisen, womit die stirnseitigen Wände auch nur wenig durch entsprechende Durchbrechungen geschwächt werden.

Bei einem Fermenter nach der Erfindung wird weiter bevorzugt, dass Wände aus segmentierten Betonfertigteilen zusammengesetzt auf Streifenfundamenten aufsitzen. Der Aufbau Weiter Seite 4 der Offenlegungsschrift
des Fermenters mit segmentierten Betonfertigteilen bedarf zunächst keiner aufwendigen Fundamentierung, sondern lediglich der Ausbildung kostengünstiger Streifenfundamente. So können die Investitionskosten weiter gesenkt werden. Dazu tragen auch die kurze Bauzeit und eine weitgehende Wetterunabhängigkeit beim Bau weiter bei. Insbesondere wird darüber hinaus ein sehr hohe, gleich bleibende Betonqualität garantiert, die auch aggressiven Substraten und Gasen jederzeit Stand hält.

In weiterer konstruktiver Ausgestaltung des Fermenters nach der Erfindung ist vorgesehen, dass die Wände unterseitig einen L-förmig abgewinkelten Fuß aufweisen, der auf einem Streifenfundament aufsitzt. Ohne jede Verankerung kann ein solches Segment-Betonfertigteil zunächst einfach auf das Streifenfundament gestellt und ausgerichtet werden. Sind dann sämtliche Betonfertigteile korrekt positioniert, ggfls. auch verspannt, kann eine, auch mehrschichtige Bodenplatte des Fermenters durch einfaches Ausgießen des Zwischenraums zwischen den Wänden erstellt werden.

Die Verwendung von Betonfertigteilen ermöglicht es ferner in einfacher Weise, dass die Längswände sich nach oben verjüngend ausgebildet sind. Dem abnehmenden Druck durch das Substrat wird damit Rechnung getragen und kann damit Material eingespart werden.

Bei der Verwendung segmentierter Betonfertigteile kann es darüber hinaus zweckmäßig sein, dass eine Wand wenigstens eine Längsverspannung aufweist, beispielsweise nach Art von Zugankern ausgebildet. Eine mechanisch stabile Konstruktion ist so gewährleistet.

Die mechanische Stabilität des Fermenters wird weiter erhöht, wenn gegenüberliegende Betonfertigteil-Segmente der Längswände oberseitig durch einen, vornehmlich auf Zug belastbaren Zuganker und durch einen auf Druck belastbaren Balken verbunden sind. Durch die Aufteilung auf eine auf Zug belastbare und eine auf Druck belastbare Verbindung können Zuganker und Balken optimal und materialgerecht ausgebildet sein. Insbesondere ist vorgesehen, dass ein Zuganker aus einem Metall, insbesondere einem Edelstahl und ein Balken ein Holzbalken ist, der lediglich in Taschen der Wände eingelegt ist. Solches senkt die Investitionskosten weiter.

Die hohe mechanische Stabilität des Fermenters nach der Erfindung erlaubt es weiter, dass der Gärraum von einem als Gasspeicher dienenden, in einem Querschnitt halbrund ausgebildeten Dach überdeckt ist. Ein externer Gasspeicher kann so vermieden werden. Darüber hinaus bedarf es keiner schweren und teuren Betondecke, die den Gärraum oberseitig abschließt. Vielmehr ist es möglich, dass gemäß der Erfindung das Dach nach Art einer Traglufthalle ausgebildet ist, insbesondere durch eine Folie ausgebildet und betriebsmäßig demontierbar. Revisionsarbeiten werden so erheblich vereinfacht und kann beispielsweise das Langachsrührwerk, wenn nötig, problemlos beim demontierten Dach entfernt und erneuert werden. Insbesondere sind auch aufgrund dieser Maßnahmen gefährliche Arbeiten innerhalb eines abgeschlossenen Gärraums weitestgehend vermieden.

Letztlich ist der Gärraum von einem Netz überdeckt.

Ein solches Netz dient in zweckmäßiger Weise der Ansiedlung von Schwefelbakterien, die bei den Gärprozessen bei einem Fermenter der voranstehend erläuterten Art dienlich sind. Darüberhinaus kann das Netz auf den Zugankern und/oder Stempeln aufliegend eine unterseitige, membranartige Folie des Gasspeichers tragen und so ein Durchhängen derselben in den Gärraum verhindern.

Die Erfindung wird anhand der Zeichnung näher erläutert, in der lediglich ein Ausführungsbeispiel schematisch dargestellt ist. In der Zeichnung zeigt:
- Fig.1:: einen Fermenter,
- Fig.2:: eine teilweise geschnittene Draufsicht auf den Fermenter gem. Fig. 1,
- Fig.3:: einen Schnitt gemäß der Linie III in Fig.2,
- Fig.4:: einen Schnitt gemäß der Linie IV in Fig.2 und
- Fig.5:: einen Schnitt gemäß der Linie V in Fig.1.

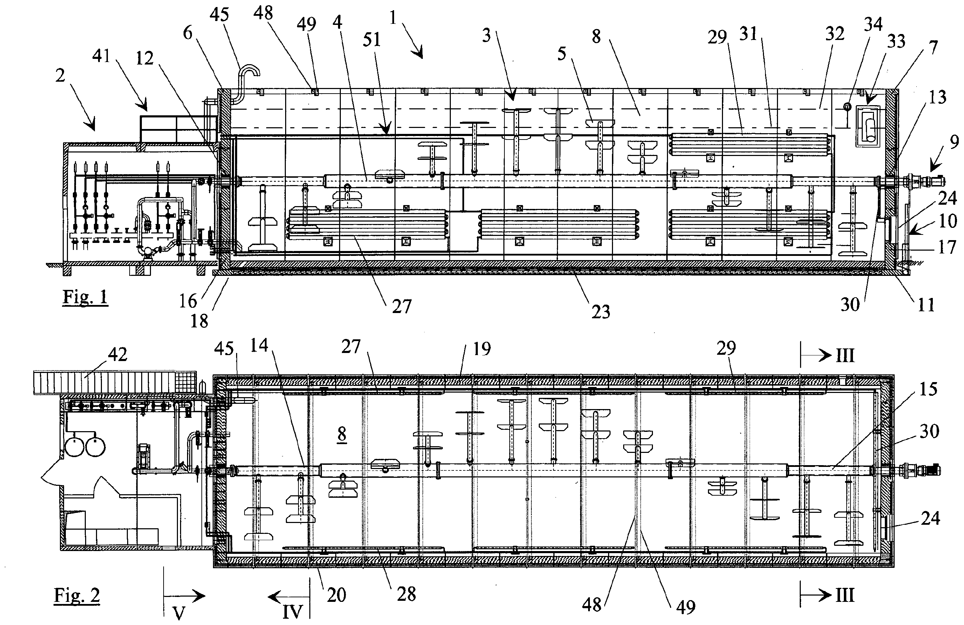

Fig.1 zeigt einen außermittigen Längsschnitt durch einen Fermenter 1 nach der Erfindung mit einem Betriebsgebäude 2, das stirnseitig an dem Fermenter 1 angeschlossen ist. Der in der Zeichnung dargestellte Fermenter 1 ist als Propfenstromfermenter ausgelegt mit einem Langachs-Rührwerk 3, das an einer zentralen Welle 4 eine Vielzahl von axial spiralförmig umlaufenden Schaufeln 5 aufweist. Die Welle 3 des Rührwerks 3 ist ausschließlich in den beiden gegenüberliegenden, stirnseitigen Wänden 6,7 gelagert. Auf eine Mittellagerung ist bewusst verzichtet.

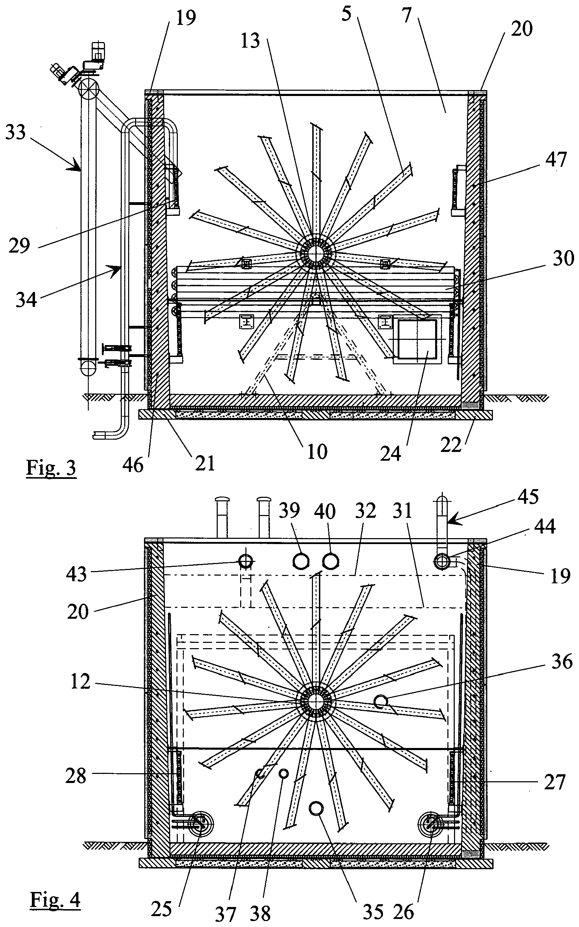

Die Welle 4 ist auf der dem Betriebsgebäude 2 gegenüberliegenden Seite durch die Wand 7 geführt und außerhalb eines Gärraums 8 an einen elektromotorischen Antrieb 9 angeschlossen. Von der stirnseitigen Wand 7 des Gärraums 8 entkoppelt wird das von dem Antrieb 9 hervorgerufene Drehmoment über eine dreieckige Stützkonstruktion 10, vergleiche auch Fig.3, in ein Fundament 11 eingeleitet.

Die in den Lagern 12,13 in den Wänden 6,7 geführte Welle 3 ist in fünf axiale Abschnitte unterteilt, von denen die stirnwandseitigen Abschnitte 14,15 gegenüber den drei mittleren Abschnitten einen reduzierten Durchmesser aufweisen. Die Lager 12,13 der Welle 4, wie auch der Antrieb 9, sind von außerhalb des Gärraums 8 zugänglich und austauschbar.

Fig.1 ist ferner entnehmbar, dass die stirnseitigen Wände 6,7, über ihre Höhe im wesentlichen von gleich bleibender Materialstärke, jeweils durch zumindest drei horizontal untergliederte Betonfertigteile ausgebildet werden. Die untersten Betonfertigteile 16,17 ruhen, bevorzugt auf L-förmig ausgebildeten Füßen, auf Streifenfundamenten 11,18.

Vergleichbar den Stirnseitigen Wänden 6,7 sind die Längswände 19,20 gleichfalls horizontal und vertikal segmentiert durch Betonfertigteile ausgebildet, wobei die untersten Reihen der Betonfertigteile gleichfalls auf Streifenfundamenten 21,22 ruhen. Sind, zumindest die untersten Reihen der Betonfertigteile positioniert auf die Streifenfundamente 11,18,21,22 aufgesetzt, kann ein mehrschichtiger Boden 23 die Wände 6,7;19,20 verbindend eingebracht werden.

Die stirnseitigen Wände 6,7 weisen über die Höhe einen im Wesentlichen gleich bleibenden Querschnitt auf. Entsprechend den stirnseitigen Wänden 6,7 sind auch die Längswände 19,20 den Gärraum 8 abschließend vergleichsweise massiv gefertigt und, nach außen hin, eine von einer Platte überdeckte Dämmschicht aufweisend.

Während die stirnseitige Wand 7 neben einer Durchbrechung zur Aufnahme des Lagers 13 lediglich noch eine Durchbrechung 24 für ein Mannloch aufweist, ist die Wand 6 mit einer Vielzahl weiterer Durchbrechungen versehen, durch die unterschiedlichste Leitungssysteme in den Betriebsraum 2 geführt sind. So dienen Durchbrechungen 25,26, bevorzugt durch Faserzementhülsen ausgekleidet, der Durchführung von Zu- bzw. Rückläufen einer Vielzahl von an den Längswänden 19,20 und der stirnseitigen Wand 7 angeordneten Heizungen 27 bis 30, die über durch die Wand 6 hindurch ins Freie geführte Rohrleitungen 51 entlüftet werden können.

Auch erhöht angebrachte Heizungen 29 liegen unterhalb eines minimalen Pegels 31 des eingebrachten Substrats, strichpunktiert angedeutet. Der obere Pegel 32, ebenfalls strichpunktiert markiert, liegt über den nach oben weisenden Schaufeln 5 des Rührwerks 3.

Eingebracht wird die Biomasse über eine Zuführung 33 in der Längswand 19. In unmittelbarer Nachbarschaft erfolgt über eine weitere Zuführung 34 auch ein Eintrag von Flüssigsubstrat. Entsprechend andernends des Fermenters 1 ist ein unterer Substratablauf 35 und ein oberer Substratablauf 36 in der Wand 6 vorgesehen.

Weitere Durchbrechungen 37,38 in der Wand 6 erlauben Füllstands- und/oder Temperaturmessungen. Klarsichtig verglaste Durchbrechungen 39,40 erlauben ferner den Blick in den Gärraum 8 von einer Arbeitsbühne 41 auf dem Dach des Betriebsgebäudes 2, die über eine Treppe 42 zugänglich ist.

Eine weitere Durchbrechung 43 dient einer Unter- bzw. Überdrucksicherung, während eine weitere, oberhalb des oberen Pegels 32 ausgebildete Durchbrechung 44 der Durchführung eines Rohrsystems 45 für die Gasentnahme dient.

Die Längswände 19,20 sind nach oben hin sich im Querschnitt verjüngend ausgebildet, vergleiche Figuren 3 und 4. Um eine gute mechanische Aussteifung der Betonfertigteile zu erhalten, weisen beide Längswände 19,20 eine Vielzahl von Längsverspannungen 46,47 auf, die beispielsweise nach Art eines Zugankers jeweils ausgebildet die Seitenwände 19,20 längs verspannen.

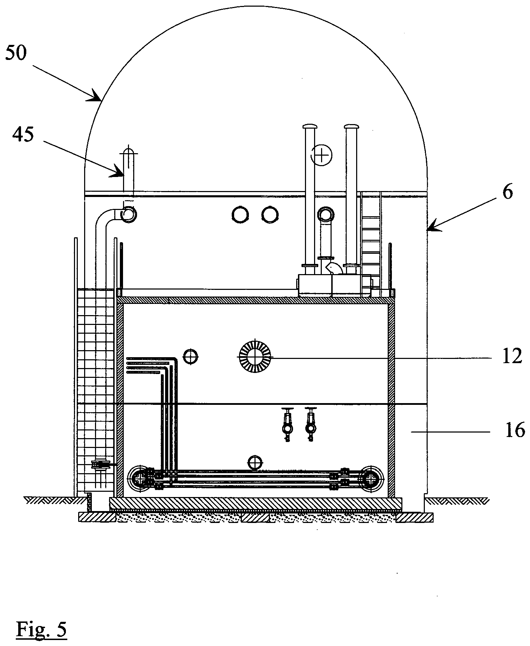

Gegenüberliegende Betonfertigteil-Segmente der beiden Längswände 19,20 sind ferner oberseitig mit einem, vornehmlich auf Zug belastbaren Zuganker 48 aus einem Metall, insbesondere aus einem Edelstahl, und einem auf Druck belastbaren Balken 49, einem Holzbalken, verbunden, der in Taschen der Segmente der Längswände 19,20 lediglich eingelegt ist. Damit weisen die Wände 6,7,19,20 eine ausreichende Stabilität auf, dass ein Dach 50 aufsetzbar ist. Das Dach 50 ist, vergleiche Fig.5, in einem Querschnitt halbrund ausgebildet und wird als Gasspeicher vorgesehen, aus dem über das Rohrsystem 45, oberhalb des oberen Pegels 32 des Substrats offen mündend, das in dem Gärraum 8 entstandene Gas entnommen werden kann.

Das Dach 50 ist nach Art einer Traglufthalle ausgebildet, dessen gasdichte Folie betriebsmäßig auch demontierbar ist, so dass der Gärraum 8 nahe zu frei von oben für beispielsweise Revisionsarbeiten zugänglich ist.

Zwischen dem Dach 50 und dem Gärraum 8 ist bevorzug noch weiter ein Netz vorgesehen, das als Aufwuchsfläche von Schwefelbakterien dient und damit eine effektive interne Entschwefelung ermöglicht. Darüber hinaus verhindert das Netz, aufliegend auf den Zugankern 48 und/oder den Balken 49 ein Durchhängen einer unterseitigern, membranartigen Folie des Gasspeichers in den Gärraum 8.

## Patentansprüche

1. Fermenter, insbesondere Propfenstromfermenter, mit einem Langachsrührwerk für ein Durchmischen einer auszugasenden Biomasse hoher Trockensubstanz, insbesondere eines Anteils von mehr als 40%, in einem Gärraum, bei dem eine Welle (4) des Rührwerks (3) ausschließlich in zwei gegenüberliegenden, stirnseitigen Wänden (6,7) des Gärraums (8) gelagert ist und wenigstens ein freies Ende des Rührwerks (3) außerhalb des Gärraums (8) an einen Antrieb (9) angeschlossen ist, **dadurch gekennzeichnet, dass** die zentrale Welle (4) des Rührwerks (3), aus mehreren axialen Abschnitten zusammengesetzt, stirnwandseitig jeweils einen Abschnitt (14,15) reduzierten Durchmessers aufweist und dass alle Abschnitte der Welle (4) eine Vielzahl von Schaufeln (5) aufweisen, wobei das Drehmoment des Antriebs (9) von einer aufgehenden, stirnseitigen Wand (7) des Gärraums (8) entkoppelt in ein Fundament (11) eingeleitet wird oder das Drehmoment des Antriebs über eine Drehmomentstütze von dem Lager abgesetzt in eine aufgehenden, stirnseitige Wand des Gärraums eingeleitet wird.

2. Fermenter nach einem oder mehreren der vorangehenden Ansprüche, **dadurch gekennzeichnet, dass** beide Lager (12,13) des Rührwerks (3) von außerhalb des Gärraums (8) zugänglich und austauschbar sind.

3. Fermenter nach einem oder mehreren der vorangehenden Ansprüche, **dadurch gekennzeichnet, dass** Wände (6,7;19,20) aus segmentierten Betonfertigteilen zusammengesetzt auf Streifenfundamenten (11,18;21,22) aufsitzen.

4. Fermenter nach einem oder mehreren der vorangehenden Ansprüche, **dadurch gekennzeichnet, dass** die Wände unterseitig einen L-förmig abgewinkelten Fuß aufweisen, der auf einem Streifenfundament aufsitzt.

5. Fermenter nach einem oder mehreren der vorangehenden Ansprüche, **dadurch gekennzeichnet, dass** Wände (19,20) sich nach oben verjüngend ausgebildet sind.

6. Fermenter nach einem oder mehreren der vorangehenden Ansprüche, **dadurch gekennzeichnet, dass** eine Wand (19,20) wenigstens eine Längsverspannung (46,47) aufweist.

7. Fermenter nach einem oder mehreren der vorangehenden Ansprüche, **dadurch gekennzeichnet, dass** gegenüberliegende Betonfertigteil-Segmente der Längswände (19,20) oberseitig durch einen Zuganker (48) und durch einen auf Druck belastbaren Balken (49) verbunden sind.

8. Fermenter nach einem oder mehreren der vorangehenden Ansprüche, **dadurch gekennzeichnet, dass** ein Zuganker (48) aus einem Metall ist.

9. Fermenter nach einem oder mehreren der vorangehenden Ansprüche, **dadurch gekennzeichnet, dass** ein Balken (49) ein Holzbalken ist.

10. Fermenter nach einem oder mehreren der vorangehenden Ansprüche, **dadurch gekennzeichnet, dass** der Gärraum (8) von einem als Gasspeicher dienenden, in einem Querschnitt halbrund ausgebildeten Dach (50) überdeckt ist.

11. Fermenter nach einem oder mehreren der vorangehenden Ansprüche, **dadurch gekennzeichnet, dass** das Dach (50) nach Art einer Traglufthalle ausgebildet ist.

12. Fermenter nach einem oder mehreren der vorangehenden Ansprüche, **dadurch gekennzeichnet, dass** das Dach (50) durch eine Folie ausgebildet betriebsmäßig demontierbar ist.

13. Fermenter nach einem oder mehreren der vorangehenden Ansprüche, **dadurch gekennzeichnet, dass** der Gärraum (8) von einem Netz überdeckt ist.

14. Fermenter nach einem oder mehreren der vorangehenden Ansprüche, **dadurch gekennzeichnet, dass** das Netz auf den Zugankern und/oder Stempeln aufliegend eine unterseitige, membranartige Folie des Gasspeichers trägt.

## Claims

1. A fermenter, in particular a plug flow fermenter, comprising a longitudinal axis agitator for mixing a biomass of high dry matter, in particular a proportion of more than 40%, to be outgassed in a fermentation chamber, in which a shaft (4) of the agitator (3) is mounted exclusively in two opposite end-face walls (6, 7) of the fermentation chamber (8), and at least one free end of the agitator (3) is connected outside of the fermentation chamber (8) to a drive (9), **characterized in that** the central shaft (4) of the agitator (3) is composed of multiple axial sections, has a section (14, 15) of reduced diameter on each end wall side, and all sections of the shaft (4) have a plurality of blades (5), wherein the torque of the drive (9) is introduced into a foundation (11) decoupled from a rising, end-face wall (7) of the fermentation chamber (8) or the torque of the drive is introduced via a torque support offset from the bearing into a rising, end-face wall of the fermentation tank.

2. The fermenter according to one or more of the preceding claims, **characterized in that** both bearings (12, 13) of the agitator (3) are accessible and replaceable from outside of the fermentation chamber (8) .

3. The fermenter according to one or more of the preceding claims, **characterized in that** walls (6, 7; 19, 20), composed from segmented, prefabricated concrete parts, are seated on strip footings (11, 18; 21, 22).

4. The fermenter according to one or more of the preceding claims, **characterized in that** the walls have an L-shaped, angled foot on the lower side which is seated on a strip footing.

5. The fermenter according to one or more of the preceding claims, **characterized in that** walls (19, 20) are designed tapering in the upward direction.

6. The fermenter according to one or more of the preceding claims, **characterized in that** one wall (19, 20) has at least one longitudinal bracing (46, 47).

7. The fermenter according to one or more of the preceding claims, **characterized in that** opposite prefabricated concrete part segments of the longitudinal walls (19, 20) are connected at the top by a tie rod (48) and by a compression-loadable beam (49) .

8. The fermenter according to one or more of the preceding claims, **characterized in that** a tie rod (48) is made from a metal.

9. The fermenter according to one or more of the preceding claims, **characterized in that** a beam (49) is a wooden beam.

10. The fermenter according to one or more of the preceding claims, **characterized in that** the fermentation chamber (8) is covered by a roof (50) functioning as a gas accumulator and designed as semicircular in one cross section.

11. The fermenter according to one or more of the preceding claims, **characterized in that** the roof (50) is designed according to a type of air-inflated structure.

12. The fermenter according to one or more of the preceding claims, **characterized in that** the roof (50) is formed by a film and may be operatively dismounted.

13. The fermenter according to one or more of the preceding claims, **characterized in that** the fermentation chamber (8) is covered by a net.

14. The fermenter according to one or more of the preceding claims, **characterized in that** the net rests on the tie rods and/or posts and supports a membrane-like film on the underside of the gas accumulator.

## Revendications

1. Fermenteur, notamment fermenteur à flux piston, pourvu d'un agitateur à axe longitudinal, destiné à mélanger intimement une biomasse à taux élevé, notamment d'un taux de plus de 40 % de substance sèche qui doit être dégazée dans un espace de fermentation, sur lequel un arbre (4) de l'agitateur (3) est logé exclusivement dans deux parois (6, 7) frontales opposées de l'espace de fermentation (8) et à l'extérieur de l'espace de fermentation (8), au moins une extrémité libre de l'agitateur (3) est raccordée sur un entraînement (9), **caractérisé en ce que** l'arbre (4) central de l'agitateur (3), composé de plusieurs tronçons axiaux comporte sur la face frontale respectivement un tronçon (14, 15) de diamètre réduit et **en ce que** tous les tronçons de l'arbre (4) comportent une pluralité de pales (5), désaccouplé d'une paroi (7) frontale ascendante de l'espace de fermentation (8), le couple de rotation de l'entraînement (9) étant introduit dans des fondations (11) ou par l'intermédiaire d'un support de couple de rotation, décalé par rapport au palier, le couple de rotation de l'entraînement étant introduit dans une paroi frontale ascendante de l'espace de fermentation.

2. Fermenteur selon l'une quelconque ou plusieurs des revendications précédentes, **caractérisé en ce que** les deux paliers (12,13) de l'agitateur (3) sont accessibles et interchangeables à partir de l'extérieur de l'espace de fermentation (8).

3. Fermenteur selon l'une quelconque ou plusieurs des revendications précédentes, **caractérisé en ce que** des parois (6, 7 ; 19, 20) composées de pièces préfabriquées en béton segmentées reposent sur des semelles filantes (11, 18 ; 21, 22).

4. Fermenteur selon l'une quelconque ou plusieurs des revendications précédentes, **caractérisé en ce que** sur la face inférieure, les parois comportent un pied coudé en forme de L qui repose sur une semelle filante.

5. Fermenteur selon l'une quelconque ou plusieurs des revendications précédentes, **caractérisé en ce que** des parois (19, 20) sont conçues en se rétrécissant vers le haut.

6. Fermenteur selon l'une quelconque ou plusieurs des revendications précédentes, **caractérisé en ce qu'**une paroi (19, 20) présente au moins une contrainte longitudinale (46, 47).

7. Fermenteur selon l'une quelconque ou plusieurs des revendications précédentes, **caractérisé en ce que** des segments opposés de pièces préfabriquées en béton des parois longitudinales (19, 20) sont reliés sur la face supérieure par un tirant d'ancrage (48) et par une poutre (49), susceptible d'être contrainte en pression.

8. Fermenteur selon l'une quelconque ou plusieurs des revendications précédentes, **caractérisé en ce qu'**un tirant d'ancrage (48) est en métal.

9. Fermenteur selon l'une quelconque ou plusieurs des revendications précédentes, **caractérisé en ce qu'**une poutre (49) est une poutre en bois.

10. Fermenteur selon l'une quelconque ou plusieurs des revendications précédentes, **caractérisé en ce que** l'espace de fermentation (8) est recouvert d'un toit (50) faisant office d'accumulateur de gaz, conçu avec une section transversale semi-ronde.

11. Fermenteur selon l'une quelconque ou plusieurs des revendications précédentes, **caractérisé en ce que** le toit (50) est conçu à la manière d'un dôme gonflable autoportant.

12. Fermenteur selon l'une quelconque ou plusieurs des revendications précédentes, **caractérisé en ce que** le toit (50), conçu sous la forme d'un film est opérationnellement démontable.

13. Fermenteur selon l'une quelconque ou plusieurs des revendications précédentes, **caractérisé en ce que** l'espace de fermentation (8) est recouvert d'un filet.

14. Fermenteur selon l'une quelconque ou plusieurs des revendications précédentes, **caractérisé en ce que** le filet, reposant sur les tirants d'ancrage et/ou sur des poinçons porte un film sous forme de membrane de la face inférieure de l'accumulateur de gaz.
